# EUROPEAN PATENT APPLICATION

(11) **EP 1 591 143 A1**
(43) Date of publication of application: **02.11.2005**
(21) Application number: 04009791.7
(22) Date of filing: 26.04.2004
(51) Int. Cl.: A61P 5/18, A61P 19/08, A61P 19/10, A61K 35/78, A61K 33/06, A61K 31/593, A61K 31/59

(54) **Composition comprising a combination of Calcium, Cimicifugae racemosae rhizoma and vitamin D and its use as a pharmaceutical in conditions or disorders associated with or resulting from calcium deficiency or as a nutritional supplement**

(71) Applicant: SCHAPER & BRÜMMER GMBH & CO. KG, D-38259 Salzgitter (DE)
(72) Inventor: Freudenstein, Johannes, Dr., 37181 Hardegsen (DE); Nisslein, Thomas, Dr., 37154 Northeim (DE)
(74) Representative: Kröncke, Rolf

(57) **Abstract**

The present invention relates to a composition for administration to subjects for preventing or treating conditions or disorders associated with or resulting from calcium deficiency comprising as active ingredients a preparation of Cimicifuga racemosa, calcium or calcium derivatives, and vitamin D or derivatives or precursors thereof. Said composition may preferably contain vitamin D3, derivatives or precursors thereof. The composition may be in form of a nutritional or dietary supplement. Said composition is particulary useful for the prophylaxis or treatment of osteoporosis.

## Description

### Background of the invention

### Field of the invention

Metabolic bone diseases are severely incapacitating and life-threatening illnesses. The most frequently observed are osteoporosis, osteomalacia, rickets, secondary nutritional hypoparathyroidism, Paget's disease, and fibrous osteodystrophy, all of which represent different forms of calcium deficiency diseases.

### Description of the related art

Calcium homeostasis is mainly regulated by organ systems that are capable of a rapid reaction, thereby reflecting dietary or physiologic requirements. Whereas the small intestine is the site where dietary calcium is absorbed, the kidney is critically important for calcium excretion. Under normal blood calcium concentrations, almost all of the calcium that enters glomerular filtrate is reabsorbed from the tubular system back into blood, which preserves blood calcium levels. If tubular reabsorption of calcium decreases, calcium is lost by excretion into urine.

On the other hand, a vast but slow reacting reservoir of calcium is represented by bone. An induced net resorption of bone mineral releases calcium into blood, whereas suppressing this effect allows calcium to be deposited in bone. Thus, for an optimal bone metabolism balanced levels of calcium resorption and deposition in bone is critical. This also involves normal calcium blood levels. In particular, it is essential to maintain a sufficiently high calcium blood level, thus providing sufficient amounts of calcium for bone formation.

Calcium supplements represent very effective means of counteracting imbalances in the bone and calcium metabolism. The cheapest, common supplement is calcium carbonate from limestone, or oyster shells. After oral intake, its intestinal absorption efficiency is up to 68%.

Further, Vitamin D, particularly vitamin D3, is one of the key factors responsible for increasing blood concentrations of calcium. Its most important effect is to facilitate absorption of calcium from the small intestine. In concert with parathyroid hormone, vitamin D also enhances fluxes of calcium out of bone. Parathyroid hormone, calcitonin, sex hormones, and bisphosphonates represent other possibilities for influencing calcium homeostasis. As they act however on a superior, more regulatory level, they are frequently accompanied by side effects of varying severity, which often surface only after a prolonged intake of the drug.

Hence, sufficient Calcium and Vitamin D3 intake is considered as an indisputable prerequisite for any study relating to the treatment of conditions or disorders associated with or resulting from calcium deficiency, e.g. osteoporosis. However, the function of Calcium and Vitamin D3 is more regarded as one of basic bone matrix constituent. In particular, Vitamin D3 promotes the intestinal uptake of orally applied calcium supplement, thus elevating the absorption efficiency.

Urinary corsslinked collagen represents one of the commonly known markers used for the determination of bone turnover, in particular of bone resorption in mammals. Therapeutically applied Calcium and Vitamin D3 does not induce profoundly altered excretion levels of urinary crosslinked collagen.

Osteoporosis is a disease characterized by reduced bone mass, deterioration of bone architecture, and increased bone fragility that leads to increased risk of fractures of the spine, hip and wrist. The increased loss of bone mass by bone turnover occur in association with estrogen deficiency or menopause. In fact, the estrogen deficiency associated with menopause is the most common cause of osteoporosis.

For a long time Cimicifugae racemosae rhizoma (CR, syn: Actaeae racemosae rhizoma) has been known to relieve climacteric complaints such as hot flushes and profuse sweating. Binding of constituents of extracts from CR to human and rodent estrogen receptors (ER) has been demonstrated. In earlier times these two observations next to the finding that CR decreases the secretion of the Lutenizing hormone (LH) lead to the conclusion of a mild estrogen agonistic activity as being responsible for the clinical efficacy.

In breast and endometrium tissue, ER binding was not associated with estrogen agonistic effects. In more recent years, a selective estrogen receptor modulation has been proposed to characterize the pharmacological profile of Cimicifuga racemosa. Based on this hypothesis, also bone protective properties of CR in animal models of human postmenopausal osteoporosis have been demonstrated.

The pathways of bone metabolism that were known to be influenced by CR are orchestrated by ER, thus leading to the presumption that CR might act via ER but in a tissue selective manner, thereby declaring it a "Phyto-SERM" (specific estrogen receptor modulator). The fact that one of the earliest bone metabolic parameters influenced by CR are renally excreted collagen crosslinks is in good agreement with SERM activities, namely showing bone protective properties. However, an influence of CR on calcium uptake or calcium excretion is not known.

Thus, a main problem still exist in the retention of calcium in the body, in particular in the blood allowing an intensified mineralization of the bone and cartilage.

Hence, there remains a need for compositions which facilitate calcium uptake and, additionally, reduce the excretion of Calcium via the urinary pathway, thus, allowing retention of calcium in the blood at high levels for bone formation. In particular, it is desirable to have a pharmaceutical or nutritional supplement composition which provides calcium blood levels to prevent bone resorption, i.e. bone loss, and/or allows for remineralization of the bone in conditions and disorders associated with or resulting from Calcium deficiency.

### Summary of the invention

The composition of the present invention allows for reducing the exrcetion of calcium, thus presumably faciliating an increased uptake of calcium into the blood. Hence, the composition is beneficial for bone formation and reduces bone resorption. Moreover, the composition according to the present invention prevents and/or remedies calcium deficiency in mammals, in particular in humans.

The composition of the present invention comprises as active ingredients a preparation of Cimicifuga racemosa, calcium or calcium derivatives and vitamin D or precursors thereof. Preferably CR is in the form of a herbal drug, more preferably in the form of an extract.

Moreover, in a preferred embodiment the composition according to the present invention contains vitamin D3 or precursors thereof.

In another aspect, the present invention relates to a method for preventing and/or treating conditions and/or disorders associated with or resulting from calcium deficiency. In particular, the present invention relates to the prevention or treatment of osteoporosis.

Further, the present invention relates to the use of a combination of a preparation of Cimicifuga racemosa, calcium or calcium derivatives and vitamin D or derivatives or precursors thereof for the preparation of a composition for the prevention or the treatment of conditions or disorders associated with or resulting from calcium deficiency. The use of a triple combination of a preparation of Cimicifuga racemosa, calcium and vitamin D3 is preferrred.

In another aspect, the present invention relates to a nutritional supplement or a dietary supplement. The supplement according to the present invention may be used to supplement food for humans or animals. The supplement can be used in a diet for preventing loss of bone mass, in particular in subjects which have a predisposition for the development of conditions or disorders associated with or resulting from calcium deficiency, e.g. subjects at high risk for osteoporosis or those with confirmed low bone densitiy. For example, the supplement according to the present invention may be used to supplement prophylactic or therapeutic regimens of conditions or disorders associated with or resulting from calcium deficiency.

### Short description of the figures

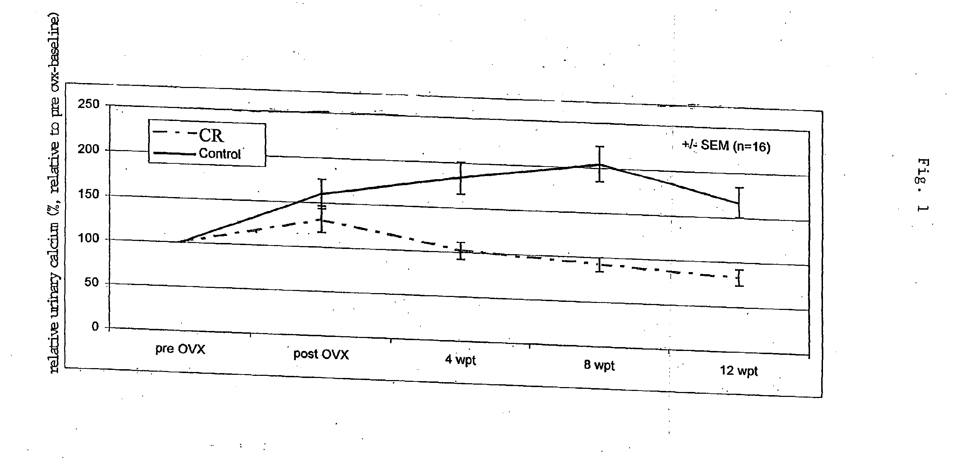

Fig. 1 demonstrates calcium excretion in animals treated with calcium and vitamin D3 alone (control) and animals receiving additional a preparation of cimifigua racemosa.

### Detailed description of the invention

The composition of the present invention provides specific and unexpected benefits. In particular the composition containing a preparation of Cimicifuga racemosa, calcium and vitamin D ensures that the amount of calcium excreted via the urinary pathway is reduced, thus, allowing calcium retention in the subject. This calcium is available for an intensified mineralization of organ systems, mainly the bone and cartilage system. Moreover, a composition containing Vitamin D, e.g. Vitamin D3, calcium and CR significantly reduces the
- mainly urinary - excretion of Calcium, when compared to administering the same dose of Calcium and Vitamin D3 alone. Any such increase in Calcium retention is beneficial in all metabolic disorders associated with or resulting from Calcium deficiency, e.g. osteoporosis.

According to the present invention, the composition contains as one of the active principle a preparation of Cimicifuga racemosa. It is preferred to use the herbal drug. It is particularly preferred to use an extract which has been prepared, for example, using an alcoholic extracting agent, particulary ethanol, isopropanol, or methanol. Preferably, the extract is prepared from the rhizome. In this connection, the effective daily dosage according to the present application is preferably a CR drug content of from 5 mg to 500 mg.

Biologically-acceptable calcium compounds include, but are not limited to, any of the well known calcium supplements, such as calcium carbonate, calcium sulfate, calcium oxide, calcium hydroxide, calcium apatite, calcium citrate-malate, bone meal, oyster shell, calcium gluconate, calcium lactate, calcium phosphate, calcium levulinate, calcium aspartate and the like. The effective daily dosage of calcium or calcium derivatives is preferably of from 200 mg to 1500 mg based on calcium.

Moreover, vitamin D or ist derivatives or precursors, also known as calciferol or its precursors, are present in the composition according to the present invention. Vitamin D includes, but is not limited to vitamin D2, vitamin D3, and the provitamins thereof.

Preferably, the inventive subject-matter contains vitamin D3 or its derivatives or precursors. The effective daily dosage of vitamin D3, its derivatives or precursors is preferably of from 50 to 500 IU vitamin D3.

The present composition may optionally contain additional vitamins and biologically acceptable minerals. Non-limiting exemplary vitamins and biologically acceptable minerals and their derivatives thereof for inclusion in the present compositions include vitamin A, B vitamins, vitamin C, vitamin E, vitamin K, folic acid, iron, magnesium, potassium, copper, chromium, zinc, molybdenum, iodine, boron, selenium, manganese, derivatives thereof or combinations thereof. These vitamins and minerals may be from any source or combination of sources, without limitation. Non-limiting exemplary B vitamins include thiamine, niacinamide, pyridoxine, riboflavin, cyanocobalamin, biotin, pantothenic aicd or combinations thereof.

Various additives may be incorporated into the present composition. Optional additives of the present composition include, without limitation, starches, sugars, fats, antioxidants, amino acids, proteins, derivatives thereof or combinations thereof.

It is also possible in the composition of the present inventive subject matter for the dosage form to combine various forms for release, which include, without limitation, immediate release, extended release, pulse release, variable release, controlled release, timed release, sustained release, delayed release, long acting, and combinations thereof. The ability to obtain immediate release, extended release, pulse release, variable release, controlled release, timed release, sustained release, delayed release, long acting characteristics and combinations thereof is performed using well known procedures and techniques available to the ordinary artisan. Each of these specific techniques or procedures for obtaining the release characteristics does not constitute an inventive aspect of this inventive subject matter all of which procedures are well known to those of ordinary skill in the art. As used herein, a "controlled release form" means any form having at least one component formulated for controlled release. As used herein, "immediate release form" means any form having all its components formulated for immediate release.

Any biologically-acceptable dosage form, and combinations thereof, are contemplated by the inventive subject matter. Examples of such dosage form include, without limitation, chewable tablets, quick dissolve tablets, effervescent tablets, reconstitutable powders, elixirs, liquids solutions, suspensions, emulsions, tablets, multi-layer tablets, bi-layer tablets, capsules, soft gelatin capsules, hard gelatin capsules, caplets, lozenges, chewable lozenges, beads, powders, granules, particles, microparticles, dispersible granules, cachets, implants, depot implants, ingestibles, injectables, infusions, health bars, confections, animal feeds, cereals, cereal coatings, foods, nutritive foods, functional foods and combination thereof. The preparation of the above dosage forms are well known to persons of ordinary skill in the art.

For example, health bars may be prepared, e.g. by mixing the formulation plus excipients (e.g., binders, fillers, flavors, colors, etc.) to a plastic mass consistency. The mass in then either extended or molded to form "candy bar" shapes that are then dried or allowed to solidify to form the final product.

Another preferred embodiment are preparations for the oral intake, like tablets. Said tablets may contain excipients or additives like binders, bulking substances, etc which are known to the artisan.

Soft gel or soft gelatin capsules may be prepared, for example, without limitation, by dispersing the formulation in an appropriate vehicle (vegetable oils are commonly used) to form a high viscosity mixture. This mixture is then encapsulated with a gelatin based film using technology and machinery known to those in the soft gel industry. The industrial units so formed are then dried to constant weight. Chewable tablets, for example, may be prepared by mixing the formulations with excipients designed to form a relatively soft, flavored, tablet dosage form that is intended to be chewed rather then swallowed. Conventional tablet machinery and procedures, that is both direct compression and granulation, i.e., or slugging, before compression, can be utilized. Those individuals involved in pharmaceutical solid dosage from production are well versed in the processes and the machinery used as the chewable dosage form is a very common dosage form in the pharmaceutical industry.
Film coated tablets, for example, may be prepared by coating tablets using techniques such as rotating pan coating methods or air suspension methods to deposit a contiguous film layer on a tablet. Compressed tablets may also be prepared by mixing the formulation with excipients.

The present inventive subject matter contemplates the use of biologically-acceptable carriers which may be prepared from a wide range of materials. Without being limited thereto, such materials include diluents, binders and adhesives, lubricants, plasticizers, disintegrants, colorants, bulking substances, flavorings, sweeteners and miscellaneous materials such as buffers and adsorbents in order to prepare a particular medicated composition.

Binders may be selected from a wide range of materials such as hydroxypropylmethylcellulose, ethylcellulose, or other suitable cellulose derivatives, acrylic and methacrylic acid co-polymers, pharmaceutical glaze, gums, milk derivatives, such as whey, starches, and derivatives, as well as other conventional binders well known to persons skilled in the art. Exemplary non-limiting solvents are water, ethanol, isopropanol, methylene chloride or mixtures and combinations thereof. Exemplary non-limiting bulking substances - fillers - include sugar, lactose, gelatin, starch, and silicon dioxide.

The plasticizers used in the dissolution modifying system are preferably previously dissolved in an organic solvent and added in solution form. Preferred plasticizers may be selected from the group consisting of diethyl phthalate, diethyl sebacate, triethyl citrate, cronotic acid, propylene glycol, butyl phthalate, dibutyl sebacate, caster oil and mixtures thereof, without limitation. As is evident, the plasticizers may be hydrophobic as well as hydrophilic in nature. Water-insoluble hydrophobic substances, such as diethyl phthalate, diethyl sebacate and caster oil are used to delay the release of water-soluble vitamins, such as vitamin B₆ and vitamin C. In contrast, hydrophilic plasticizers are used when water-insoluble vitamins are employed which aid in dissolving the encapsulated film, making channels in the surface, which aid in nutritional composition release.

The composition of the present inventive subject matter may be administered in a partial, i.e., fractional dose, one or more times during a 24 hour period, a single dose during a 24 hour period of time, a double dose during a 24 hour period of time, or more than a double dose during a 24 hour period of time. Fractional, double or other multiple doses may be taken simultaneously or at different times during the 24 hour period. That means, the composition according to the present invention may be administered simultaneously, separately or sequentially.

The compositions of the present invention are intended for use by humans and other mammals. The dosages are individually adjusted according to body weight and the condition of the subject as well as the prescribed regimen.

The present invention further relates to the use of the compositions of the present invention in the treatment of conditions or disorders associated with or resulting from a calcium deficiency. In particular, the present invention relates to the use for the treatment of osteoporosis, osteomalacia, rickets, secondary nutritional hypoparathyroidism, Paget's disease, and fibrous osteodystrophy. The treatment comprises the administration of a preparation of Cimicifuga racemosa, calcium or calcium derivatives, and vitamin D, preferably vitamin D3. The above components may be administered simultaneously, separately or sequentially.

Moreover, the present invention relates to the use of the compositions according to the present invention for prophylaxis of disorders or conditions associated with or resulting from calcium deficiency. In particular, it relates to the prophylactic treatment of subjects which have a predisposition for the development of conditions or disorders associated with or resulting from calcium deficiency, e.g. subjects at high risk for osteoporosis or those with confirmed low bone densitiy.

Further, the present invention provides for nutritional supplement or dietary supplement which may be added to food or animal food. For example, the nutritional supplement is suitable for oral intake. The amount of the nutritional or dietary supplement to be taken daily vary within broad limits, depending, for example, on the subjects's age and body weight.

The following example illustrates the efficacy of the present invention, however, it is not to be construed as limiting the inventive subject matter thereto.

### Example

32 adult female Sprague Dawley rats were kept under standard housing conditions. They were fed with a soy free diet, providing a daily intake of 450 mg Calcium and 28 IU Vitamin D3 per kg body weight (i.e. approx. 170 mg Calcium and 11 IU Vitamin D3 per per animal per day). Ovariectomy (OVX) was performed at approx. 12 months of age.

Starting at day 14 after OVX, 16 animals were treated via gavage with a daily dose of 300 *µ*l of an isopropanolic/aqueous extract from CR per kg body weight, whereas the residual 16 animals were treated with the equivalent amount of isopropanol.

The daily dose of 300 µl liquid extract per kg body weight provides for a daily uptake of approx. 60 mg herbal drug per day, corresponding to 100 times human therapeutics dose. The liquid extract used in this experiment originated from the regular production process and was part of a batch destined for tablet production. It was manufactured by adding 1.2 parts of isopropylic alcohol (40% v/v) to 1 part of ground herbal drug (black cohosh rootstock) and subjecting it to a maceration process of 7 days, thus guaranteeing a drug-extract of 0.78-1.14:1.

Compared to urinary calcium excretion before ovariectomy (OVX), animals that had been treated with CR for 8 weeks showed a 32% decreased relative urinary calcium level (levelled to creatinine).

Calcium measurements were conducted on individual acidified 24-h-urine samples. Calcium was measured in a commercialy available test kit using the arsenazo III method. Arsenazo III, when complexed with calcium turns purple, the intensity of colouring being proportional to the amount of calcium. Calcium excretion was corrected for creatinine, which was measured via HPLC according to standard methods (Ambrose et al. 1983, Clin Chem 29: 256-259).

Compared to animals that had for the same period of time been treated with the vehicle control of the CR preparation, we observed a statistically significant reduction in absolute urinary calcium excretion from 3.0 +/- 0.2 mg/24 h to 2.3 +/- 0.2 mg/24 h.

Animals of both groups excreted comparable amounts of Calcium via their faeces. This amounted to 144 +/- 2,65 mg/24 h without showing any significant inter group differences. An example of the results is provided in figure 1.

Calcium is mainly administered in combination with Vitamin D3, which augments the resorption and distribution of Calcium. Calcium is resorbed from the gastrointestinal tract and mainly renally excreted. The above data demonstrates that a fixed combination with CR significantly reduces the urinary excretion of concomitantly applied Calcium.

As the supply of Vitamin D3 did not differ between experimental groups, it is concluded that the described observation demonstrates the therapeutic effect of a triple-combination, where a defined amount of CR is included into a fixed combination of calcium and Vitamin D3. As the excretion of Calcium with faeces has not been altered it may be assumed that Cimicifuga does not influence Calcium resorption by an additional Vitamin D like effect.

Decreased levels of urinary Calcium can be interpreted as an increase in Calcium retention. The responsible mechanisms is still unknown. An increase in Calcium retention predominantly comes to bear in mineralized organ systems, mainly bone, which is beneficial to prevent or to cure calcium deficiencies.

## Claims

1. A pharmaceutical composition comprising as active ingredients a preparation of Cimicifuga racemosa, calcium or calcium derivatives, and vitamin D or derivatives or precursors thereof for administration to subjects for preventing or treating conditions or disorders associated with or resulting from calcium deficiency.

2. A composition according to claim 1 wherein said composition contains vitamin D3 or derivatives or precursors thereof.

3. Composition according to claim 1 or claim 2 wherein said composition additionally contains filler, diluents, binders and adhesives, lubricants, plasticizers, disintegrants, colorants, bulking substances, flavorings, and/or sweeteners.

4. Composition according to any one of claims 1 to 3 wherein the preparation of Cimicifuga racemosa is in a form of a herbal drug.

5. Composition according to any one of claims 1 to 3 wherein the preparation of Cimicifuga racemosa is in a form of an extract.

6. Composition according to any one of claims 1 to 5 being in form of granules, tablets, solutions, and dispersions.

7. A nutritional supplement or dietary supplement for preventing or treating conditions or disorders associated with or resulting from calcium deficiency comprising as active ingredients a preparation of Cimicifuga racemosa, calcium or calcium derivatives, and vitamin D or derivatives or precursors thereof.

8. A nutritional or dietary supplement according to claim 7 containing vitamin D3 or derivatives or precursors thereof.

9. A nutritional or dietary supplement according to claim 7 or claim 8, wherein the preparation of Cimicifuga racemosa is in a form of a herbal drug.

10. A nutritional or dietary supplement according to claim 7 or claim 8, wherein the preparation of cimicifuga racemosa is in a form of an extract.

11. A nutritional or dietary supplement according to anyone of claims 7 to 10 being in form of tablets, bars, powder, granules or capsules.

12. Use of a preparation according to any one of claim 1 to 11 as an additve for food or animal food.

13. Use of a preparation according to any one of claims 1 to 6 for the preparation of a pharmaceutical for preventing or treating conditions or disorders associated with all resulting from calcium deficiency.

14. The use according to claim 13 for the treatment of osteoporosis, osteomalacia, rickets, secondary nutritional hypoparathyroidism, Paget's disease, and fibrous osteodystrophy.

15. Pharmaceutical composition according to any one of claims 1 to 6 comprising a preparation of cimicifuga racemosa in a daily dosage of from 5 mg to 500 mg drug, calcium or calcium derivative in a daily dosage of from 200 mg to 1.500 mg based on calcium, and vitamin D3, its derivatives or precursors in a daily dosage of from 50 to 500 IU vitamin D3.
